**Europäisches Patentamt**

**European Patent Office**

**Office européen des brevets**

(19)

(11) Veröffentlichungsnummer: **0 116 298**
**B1**

(12) # EUROPÄISCHE PATENTSCHRIFT

(45) Veröffentlichungstag der Patentschrift:
**03.06.87**

(21) Anmeldenummer: **84100290.0**

(22) Anmeldetag: **12.01.84**

(51) Int. Cl.⁴: **A 61 L 31/00**, A 61 F 2/38,
C 04 B 35/46

(54) **Keramischer Knochenersatzwerkstoff und Verfahren zu seiner Herstellung.**

(30) Priorität: **14.01.83 DE 3301122**

(43) Veröffentlichungstag der Anmeldung:
**22.08.84 Patentblatt 84/34**

(45) Bekanntmachung des Hinweises auf die Patenterteilung:
**03.06.87 Patentblatt 87/23**

(84) Benannte Vertragsstaaten:
**AT BE CH DE FR GB IT LI NL SE**

(56) Entgegenhaltungen:
**DE - A - 2 659 591**
**US - A - 3 787 900**
**US - A - 3 905 047**

**Chemical Abstracts, Band 99, Nr. 26, 26. Dezember 1983,
Columbus, Ohio, USA; J.F. OSBORN et al. "Calcium
phosphate-titanium dioxide ceramic. Fabrication and
material tests of a new implant material", Seite 381,
Spalte 1, Abstract Nr. 218549b**

(73) Patentinhaber: **Osborn, Johannes Friedrich, Dr.,
Innocentiastrasse 5, D-2000 Hamburg 13 (DE)**

(72) Erfinder: **Osborn, Johannes Friedrich, Dr.,
Innocentiastrasse 5, D-2000 Hamburg 13 (DE)**

(74) Vertreter: **Betzler, Eduard, Dipl.-Phys. et al,
Postfach 70 02 09 Plinganserstrasse 18a,
D-8000 München 70 (DE)**

## Beschreibung

Die Erfindung betrifft einen keramischen Knochenersatzwerkstoff mit bioaktiven Eigenschaften auf der Grundlage von Hydroxylapatiten und richtet sich ferner auf ein Verfahren zur Herstellung eines solchen Knochenersatzwerkstoffes.

Die günstigen biologischen Eigenschaften von Hydroxylapatiten und einigen Tricalciumphosphatkeramiken sind seit längerer Zeit bekannt (DE-A- 26 59 591). Dabei sind von beiden Werkstoffgruppen jeweils im Körpermilieu verhältnismässig stabile oder auch im Körpermilieu mehr oder weniger schnell resorbierbare Versionen bekannt. Diese bekannten Hydroxylapatite und Tricalciumphosphatkeramiken sind in ihrer Anwendbarkeit als Knochenersatzwerkstoffe jedoch durch ihre schwer reproduzierbare und verhältnismässig geringe mechanische Festigkeit in ihren Anwendungsmöglichkeiten begrenzt. Zur Überwindung dieser Schwierigkeiten sind schon vielfältige Versuche unternommen worden, derartige Keramiken als Schichten auf mechanisch ausreichend festen Werkstoffen aufzubringen. Alle derartigen Versuche haben bisher noch nicht zu brauchbaren Knochenersatzwerkstoffen geführt. Die aufgebrachten Schichten erwiesen sich meist nicht haftfest genug oder wurden vom Gewebe allmählich aufgelöst. Es ist ausserdem ein Versuch bekannt geworden, derartige Apatitpulver mit Titanpulvern und gesintertem Titan zusammen zu verarbeiten (K. Asano, T. Ichiko und H. Tonegawa, «development of hydroxylapatitetitanium composite material as an implant material, 8th annual meeting of the society for biomaterials, Orlando, Walt Disney World, Fla., USA 24. 27. April 1982). Die gemeinsame Verarbeitung von Titanpulver mit Hydroxylapatit führt jedoch, wie wir festgestellt haben bei Ausbildung verschiedener, zum Teil keineswegs besonders körperverträglichen Phosphatphasen.

Es ist zwar ein Knochenersatzwerkstoff bekannt (US-PS 3 787 900), bei dem Spinell Verwendung findet, und zwar zur Bildung diskreter Mikrokristalle neben der Bildung diskreter Mikrokristalle aus Calciumphosphatverbindungen. Der Austausch Spinell gegen Rutil ist jedoch dem Fachmann nicht ohne weiteres nagelegt, da beim bekannten Knochenersatzwerkstoff Rutil als Zusatzwerkstoff vorgesehen ist, der nur bedarfsweise für bestimmte Herstellverfahren zum Zwecke einer Wärmekonsolidierung eingesetzt wird, und zwar in Mengen von weniger als 5 Gew.-%. Rutil dient hierbei als sogenannter Mineralbildner und ist im übrigen als eine Alternative unter zahlreichen anderen Alternativen von Zusatzwerkstoffen genannt.

Der Erfinder hatte sich die Aufgabe gestellt, einen keramischen Knochenersatzwerkstoff zu synthetisieren, der die günstigen oben erwähnten biologischen Eigenschaften der Hydroxylapatitkeramiken und Tricalciumphosphatkeramiken aufrecht erhalten, aber gleichzeitig eine höher mechanische Festigkeit in reproduzierbarer Weise einzustellen gestattet.

Die Lösung dieser Aufgabe wird dadurch erreicht, dass das Ausgangsmaterial aus 70 bis 95 Gew.-% Titanoxid- und der Rest aus Hydroxylapatitpulver besteht und das vorverdichtete Gemisch der Pulver in einer Wärmebehandlung in eine mehrphasige, polykristalline Keramik überführt worden ist, welche eine Druckfestigkeit von mehr als $1000 \, N/mm^2$, eine Biegebruchfestigkeit von mehr als $110 \, N/mm^2$ und eine Dichte von mehr als $3,6 \, g/cm^3$ bei Zimmertemperatur aufweist.

Ausgestaltungen dieser Lösung und ein Verfahren zur Herstellung eines erfindungsgemässen Werkstoffes sind in den Unteransprüchen im einzelnen dargelegt.

Die auf die beschriebene Art und Weise hergestellten keramischen Knochenersatzwerkstoffe weisen ein heterogenes, mindestens zwei Phasen enthaltendes Gefüge auf. Es besteht einerseits aus Rutilpartikeln, die sich aus den ursprünglich eingesetzten Titandioxidpulvern gebildet hatten, zum anderen aus β-Whitlockit, der sich aus dem eingesetzten Hydroxylapatitpulver gebildet hat. Dabei liegt das β-Whitlockit im wesentlichen als Korngrenzsubstanz zwischen den Rutilkörnern.

Der hier schon mehrfach benutzte Begriff bioaktiv bezieht sich auf Werkstoffe, die nach Einbringen in knöcherne Strukturen die Anlagerung von Knochengewebe längs ihrer Oberfläche offensichtlich begünstigen, wie mehrfach in der Vergangenheit festgestellt worden ist. Der Begriff bioaktiv in diesem Sinne ist zu verstehen im Gegensatz zu bioniert. Als bioniert werden Werkstoffe bezeichnet, die im Körpermilieu und insbesondere auch in Knochenumgebung die dort ablaufenden biologischen Reaktionen nicht beeinflussen.

Zur Herstellung des erfindungsgemässen Knochenersatzwerkstoffes dienen im übrigen mehrere Verfahrensweisen der keramischen Technologie. So können für das Mischen und die Formgebung alle in der keramischen Technologie bekannten Vorgehensweisen benutzt werden. Auch ein z.B. isostatisches Nachverdichten der bereits kompaktierten Teile ist möglich. Die Teile können in dem kompaktierten aber noch nicht gebrannten Zustand auch noch mechanisch bearbeitet werden, falls das im Interesse der Formgebung erwünscht ist. Die Wärmebehandlung kann durchaus an Normalatmosphäre erfolgen, für spezielle Zwecke könnte es auch nützlich sein, eine Schutzgasatmosphäre zu benützen. Ausserdem ist es möglich die Schritte der Wärmebehandlung und der Verdichtung in einem Arbeitsgang zusammenzufassen.

Die erfindungsgemässen keramischen Knochenersatzwerkstoffe verhalten sich in biologischem Milieu, insbesondere nach Einbringen in knöcherne Umgebung genauso wie nicht titandioxidhaltige bioaktive Calciumphosphatkeramiken. Sie weisen jedoch, darauf sei nochmals hingewiesen, eine höhere Festigkeit bei besserer Reproduzierbarkeit der mechanischen Eigenschaften auf.

Bezüglich der Wärmebehandlung ist darauf hingewiesen, dass diese eine Temperaturerhöhung auf 1250°C bis 1450°C umfasst, wobei diese Endtemperatur für eine Zeit zwischen 5 und 25 Stunden zu halten ist und die höhere Temperatur einer kürzeren, die niedrigere Temperatur einer längeren Haltezeit zuzuordnen ist.

## Patentansprüche

1. Keramischer Knochenersatzwerkstoff mit bioaktiven Eigenschaften auf der Grundlage von Hydroxylapatit, dadurch gekennzeichnet, dass das Ausgangsmaterial aus 70 bis 95 Gew.-% Titanoxid- und der Rest aus Hydroxylapatitpulver besteht, und das vorverdichtete Gemisch der Pulver in einer Wärmebehandlung in eine mehrphasige, polykristalline Keramik überführt worden ist, welche eine Druckfestigkeit von mehr als 1000 N/mm², eine Biegebruchfestigkeit von mehr als 110 N/mm² und eine Dichte von mehr als 3,6 g/cm³ bei Zimmertemperatur aufweist.

2. Knochenersatzwerkstoff nach Anspruch 1, dadurch gekennzeichnet, dass die Keramik neben Rutil β-Whitlockit enthält.

3. Knochenersatzwerkstoff nach Anspruch 1 oder 2, dadurch gekennzeichnet, dass die Pulver eine maximale Korngrösse von 30 μm aufweisen.

4. Verfahren zur Herstellung eines Knochenersatzwerkstoffes nach den Ansprüchen 1 bis 3, dadurch gekennzeichnet, dass ein Gemisch aus 70 bis 95 Gew.-% Titanoxidpulver mit Rest Hydroxylapatitpulver vorverdichtet und einer Wärmebehandlung zur Überführung in eine mehrphasige polykristalline Keramik unterworfen wird.

5. Verfahren nach Anspruch 4, dadurch gekennzeichnet, dass das Vorverdichten mit einem Druck von mindestens 5000 N/cm² erfolgt.

6. Verfahren nach Anspruch 4 und/oder 5, dadurch gekennzeichnet, dass die Wärmebehandlung eine Temperaturerhöhung bis zwischen 1250 bis 1450°C für 25 bis 5 Stunden einschliesst.

## Claims

1. A ceramic bone substitute material with bioactive properties on the basis of hydroxyl apatite, characterized in that the starting material consists of 70 to 95 wt.% of titanic oxide powder, the remainder being hydroxyl apatite powder, and the precompressed mixture of the powders has been converted in a heat treatment to a multiphase, polycrystalline ceramic material which has a resistance to pressure of more than 1000 N/mm², a bending strength of more than 110 N/mm² and a density of more than 3.6 g/cm³ at room temperature.

2. A bone substitute material according to claim 1, characterized in that the ceramic material contains not only rutile but also β-whitlockite.

3. A bone substitute material according to claim 1 or 2, characterized in that the powders have a maximum grain size of 30 μm.

4. A method for producing a bone substitute material according to claims 1 to 3, characterized in that a mixture of 70 to 95 wt.% of titanic oxide powder with the remainder being hydroxyl apatite powder is precompressed and subjected to a heat treatment for conversion into a multiphase polycrystalline ceramic material.

5. A method according to claim 4, characterized in that the precompression is performed at a pressure of at least 5000 N/cm².

6. A method according to claim 4 and/or 5, characterized in that the heat treatment includes a temperature increase up to between 1250 and 1450°C for 25 to 5 hours.

## Revendications

1. Matière céramique pour le remplacement des os ayant des propriétés bioactives et réalisée à base d'hydroxy-apatite, caractérisée en ce que la matière de départ est constituée par 70 à 95% en poids d'oxyde de titane, le reste étant de la poudre d'hydro-apatite, et que le mélange des poudres précomprimé est transformé, par traitement thermique, en une matière céramique polycristalline à plusieurs phases qui présente une résistance à la compression de plus de 1000 N/mm², une résistance à la flexion de plus de 110 N/mm² et une densité de plus de 3,6 g/cm² à la température ordinaire.

2. Matière de remplacement des os selon la revendication 1, caractérisée en ce que la matière céramique contient, à côté du rutile, de la whitlockite β.

3. Matière de remplacement des os selon l'une des revendications 1 ou 2, caractérisée en ce que les poudres ont une granulométrie maximale de 30 μm.

4. Procédé de fabrication d'une matière de remplacement des os selon l'une des revendications 1 à 3, caractérisé en ce qu'un mélange de 70 à 95% en poids de poudre d'oxyde de titane et d'un reste constitué par de la poudre d'hydroxyapatite est précomprimé puis soumis à un traitement thermique pour sa transformation en une matière céramique polycristalline à plusieurs phases.

5. Procédé selon la revendication 4, caractérisé en ce que la précompression s'effectue sous une pression d'au moins 5000 N/cm².

6. Procédé selon l'une des revendications 4 ou 5 (ou les deux), caractérisé en ce que le traitement thermique comporte une élévation de la température jusqu'à une valeur comprise entre 1250 et 1450°C pendant 25 à 5 heures.